# EUROPEAN PATENT APPLICATION

(11) **EP 4 840 936 A1**
(43) Date of publication of application: **18.11.2026**
(21) Application number: 24916925.1
(22) Date of filing: 24.12.2024
(51) Int. Cl.: C12M 1/42, A01H 5/00, C12M 1/00, C12N 5/04, C12N 5/10, C12N 13/00, C12N 15/89

(54) **METHOD FOR INTRODUCING SUBSTANCE INTO PLANT CELL, GENETICALLY MODIFIED PLANT OR PLANT CELL AND METHODS FOR PRODUCING SAME, AND PROGRAM FOR CONTROLLING DEVICE FOR INTRODUCING SUBSTANCE INTO PLANT CELL**

(30) Priority: 11.01.2024 JP 2024002862; 29.08.2024 JP 2024147969
(71) Applicant: Yokogawa Electric Corporation, Musashino-shi, Tokyo 180-8750 (JP)
(72) Inventor: NAGO, Shiho, Musashino-shi, Tokyo 180-8750 (JP); KUSAURA, Hikari, Musashino-shi, Tokyo 180-8750 (JP); KIUCHI, Takanobu, Musashino-shi, Tokyo 180-8750 (JP); KAMACHI, Fumitaka, Musashino-shi, Tokyo 180-8750 (JP); OHATA, Takatoshi, Musashino-shi, Tokyo 180-8750 (JP)
(74) Representative: Stöckeler, Ferdinand
(86) International application number: PCT/JP2024/045795
(87) International publication number: WO 2025/150423

(57) **Abstract**

Provided is an automatically controlled system for injection into plant cells under conditions specialized for plants. A method for introducing a substance into a plant cell includes a) positioning a nanopipette at a position corresponding to a cell, b) moving the nanopipette in a cell direction to a predetermined position based on current between the inside of the nanopipette and an electrolytic solution, c) moving the nanopipette in the cell direction by a set penetration distance to penetrate the cell, d) applying a voltage to eject the substance into the cell, and e) retracting the nanopipette.

## Description

### TECHNICAL FIELD

The present disclosure relates to a method for introducing a substance into a plant cell, a genetically modified plant or plant cell and a method for producing the same, and a control program for an apparatus for introducing a substance into a plant cell.

### BACKGROUND

Genome editing tools such as the CRISPR-Cas9 system are expected to be readily used for genetic modification of individual organisms. In particular, methods that may be considered for introducing genome editing tools into individual plants include, for example, an injection method using a nanopipette described in Patent Literature (PTL) 1 and 2, and a method for introducing a complex of gold particles and a genome editing tool into an individual plant using a particle gun method described in PTL 3 and 4 (iPB (in planta Particle Bombardment) method). With the iPB method, the cells into which the genome editing tool is introduced are random, and damage may occur to the introduced cells or the introduced substance, raising concerns about the stability of gene modification efficiency and its invasiveness to the individual plant. Injection methods using nanopipettes are expected to improve the stability of gene modification efficiency and to reduce invasiveness, but injection conditions specific to plants have not yet been established.

### CITATION LIST

### Patent Literature

PTL 1: WO 2014/160036 A1
PTL 2: WO 2013/012452 A1
PTL 3: JP 2017-205103 A
PTL 4: JP 2017-205104 A

### SUMMARY

### (Technical Problem)

The present disclosure aims to establish injection conditions specific to plants in a method for injecting into a plant cell using a nanopipette, and to provide an automatically controlled injection system for plant cells.

### (Solution to Problem)

[1] A method for introducing a substance into a plant cell, the method comprising:
   a) positioning a nanopipette filled with a substance at a position corresponding to a plant cell in an electrolytic solution;
   b) measuring a current between an inside of the nanopipette and the electrolytic solution and moving the nanopipette in a cell direction to a position at which a drop rate from a steady state current is a set current drop rate of 2 % or more and 50 % or less;
   c) moving the nanopipette in the cell direction by a set penetration distance of 1 µm or more and 50 µm or less to penetrate the plant cell;
   d) applying a voltage between the inside of the nanopipette and the electrolytic solution to eject the substance into the plant cell; and
   e) retracting the nanopipette.
[2] The method according to [1], wherein the substance is a protein; a mixture or complex containing a protein; a mixture or complex containing a protein and a nucleic acid; a nucleic acid; or a dye.
[3] The method according to [2], wherein the substance is a substance for genome editing.
[4] The method according to [2], wherein
   the substance is a positively charged substance or a negatively charged substance,
   in a case in which the substance is a positively charged substance, d) is performed by applying a voltage so that the inside of the nanopipette is at a positive potential and the electrolytic solution is at a negative potential, and
   in a case in which the substance is a negatively charged substance, d) is performed by applying a voltage so that the inside of the nanopipette is at a negative potential and the electrolytic solution is at a positive potential.
[5] The method according to [4], wherein
   d) is performed by applying a voltage at a set applied voltage of -11 V or more and +11 V or less and a set application time of 0.1 seconds or more and 10.0 seconds or less.
[6] A method for producing a genetically modified plant or plant cell, the method comprising:
   a) positioning a nanopipette filled with a genetic modification substance at a position corresponding to a plant cell in an electrolytic solution;
   b) measuring a current between an inside of the nanopipette and the electrolytic solution and moving the nanopipette in a cell direction to a position at which a drop rate from a steady state current is a set current drop rate of 2 % or more and 50 % or less;
   c) moving the nanopipette in the cell direction by a set penetration distance of 1 µm or more and 50 µm or less to penetrate the plant cell;
   d) applying a voltage between the inside of the nanopipette and the electrolytic solution to eject the substance into the plant cell; and
   e) retracting the nanopipette.
[7] A genetically modified plant or plant cell produced by a method comprising:
   a) positioning a nanopipette filled with a genetic modification substance at a position corresponding to a plant cell in an electrolytic solution;
   b) measuring a current between an inside of the nanopipette and the electrolytic solution and moving the nanopipette in a cell direction to a position at which a drop rate from a steady state current is a set current drop rate of 2 % or more and 50 % or less;
   c) moving the nanopipette in the cell direction by a set penetration distance of 1 µm or more and 50 µm or less to penetrate the plant cell;
   d) applying a voltage between the inside of the nanopipette and the electrolytic solution to eject the substance into the plant cell; and
   e) retracting the nanopipette.
[8] A control program for an apparatus for introducing a substance into a plant cell, the control program comprising instructions for executing processing comprising:
   a) positioning a nanopipette filled with a genetic modification substance at a position corresponding to a plant cell in an electrolytic solution;
   b) measuring a current between an inside of the nanopipette and the electrolytic solution and moving the nanopipette in a cell direction to a position at which a drop rate from a steady state current is a set current drop rate of 2 % or more and 50 % or less;
   c) moving the nanopipette in the cell direction by a set penetration distance of 1 µm or more and 50 µm or less to penetrate the plant cell;
   d) applying a voltage between the inside of the nanopipette and the electrolytic solution to eject the substance into the plant cell; and
   e) retracting the nanopipette.

### (Advantageous Effect)

According to the present disclosure, injection conditions specific to plants can be established in a method for injecting into a plant cell using a nanopipette, and an automatically controlled injection system for plant cells can be provided.

### BRIEF DESCRIPTION OF THE DRAWINGS

In the accompanying drawings:
FIG. 1 is a schematic diagram of a system for use with the method of the present disclosure and a pipette approach step in the method of the present disclosure;

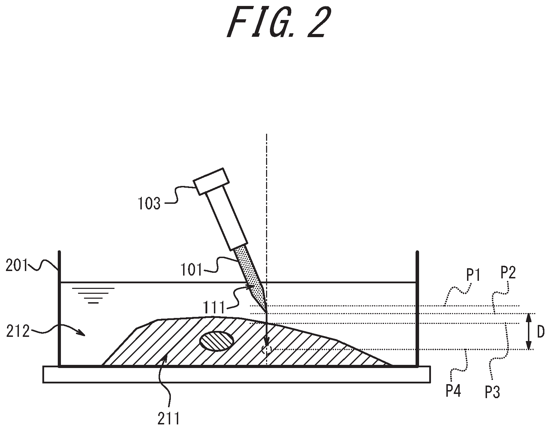

FIG. 2 is a conceptual diagram of a pipette penetration step in the method of the present disclosure;

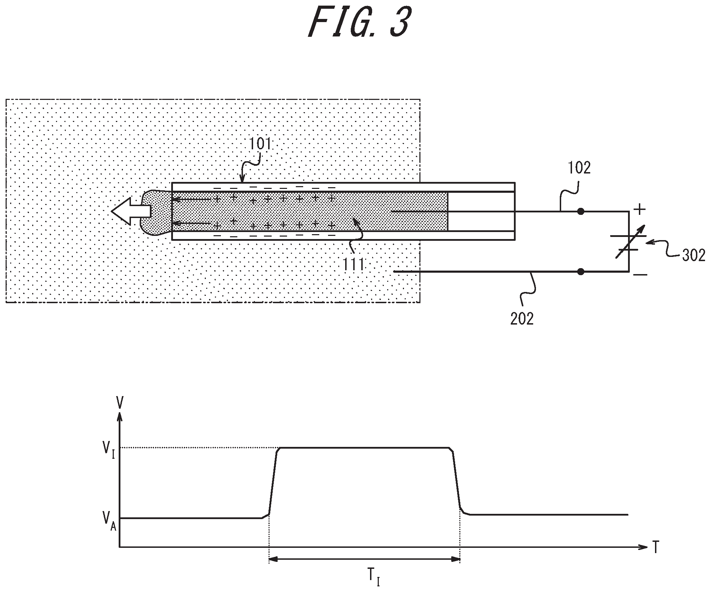

FIG. 3 is a schematic diagram of a pipette substance ejection step in the method of the present disclosure;

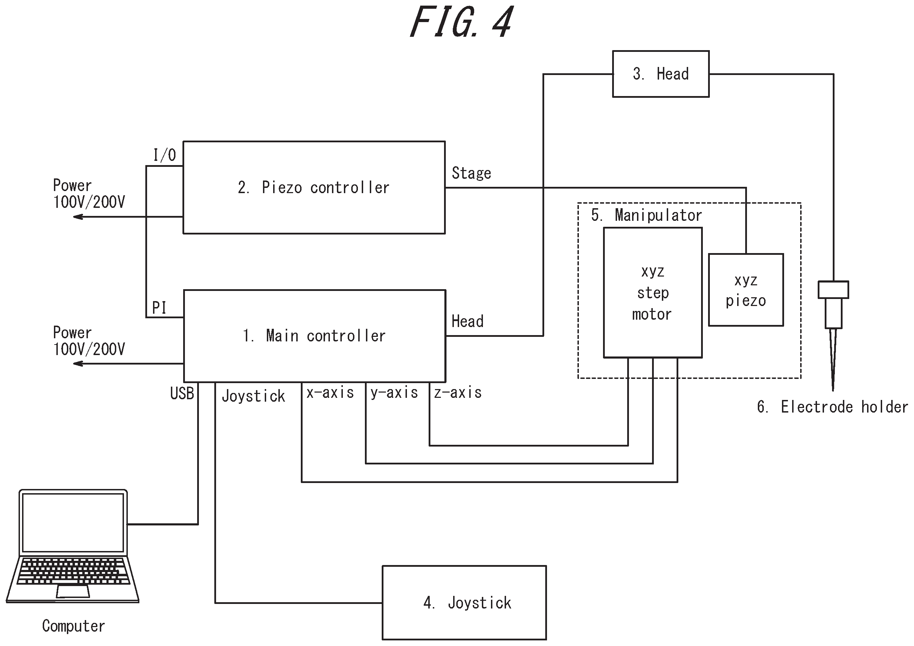

FIG. 4 is a diagram illustrating an example control mechanism of a system for use with the method of the present disclosure;
FIG. 5 illustrates photographs of rice callus subjected to genome editing using the method of the present disclosure, where the upper and lower images illustrate two different samples, respectively, and in the images on the right, the whitened areas are outlined;
FIG. 6 illustrates photographs of an individual barley plant subjected to genome editing using the method of the present disclosure, where the lower images illustrate an enlarged view of the upper images, and in the images on the right, the whitened areas are outlined; and
FIG. 7 is a graph illustrating the survival rate of barley into which a reagent solution was introduced by the method of the present disclosure according to various conditions of injection voltage.

### DETAILED DESCRIPTION

Hereinafter, the present disclosure will be described in detail with reference to the drawings as necessary. However, the drawings are merely examples for explaining the present disclosure, and the technical scope of the present disclosure is not limited by the examples in the drawings.

### [System for introducing substance into a plant cell]

A system (system for introducing a substance into a plant cell, hereinafter referred to as the "system of the present disclosure") for use in the methods of the present disclosure (method for introducing a substance into a plant cell and method for producing a genetically modified plant cell) is described. The system of the present disclosure includes, for example, the following components:
- a nanopipette;
- a three-dimensional (also referred to as "xyz" for convenience) movement pipette holder for moving the nanopipette in three dimensions (xyz);
- an electrode capable of contacting a liquid filled inside the nanopipette (hereinafter referred to as the "pipette electrode");
- an electrode capable of contacting an electrolytic solution in a cell holder (hereinafter referred to as the "reference electrode");
- a current measurement circuit for measuring the current between the pipette electrode and the reference electrode; and
- a voltage application circuit for applying a voltage between the pipette electrode and the reference electrode.

Additionally, the system of the present disclosure may further include a position input device for moving a three-dimensional movement module by manual input. The system of the present disclosure is installed on an optical microscope for use. In addition, the system of the present disclosure is used by placing the cell holder on the optical microscope, the cell holder holding plant cells and an electrolytic solution in which the plant cells are immersed, and by holding, inside the nanopipette, a solution containing a substance to be introduced into the plant cells.

Each component will be described below.

### (Nanopipette)

The term "nanopipette" refers to a tubular structure having a nanoscale tip opening. A nanoscale tip opening is, for example, a conical tip opening (i.e., a nanopore) of about 10 nm to about 500 nm, preferably about 50 nm (±20 %). The material used for the nanopipette is an inert, non-biological material, such as glass or quartz. The inner wall of the nanopipette may be surface-treated to suppress adsorption of substances (for example, nucleic acids, proteins) filled inside the nanopipette. The nanopipette preferably has a shape or scale that allows an electrode to be inserted into the nanopipette so as to contact the solution within the nanopipette.

Nanopipettes have a single channel (also called a "barrel" or "bore") or a plurality of parallel channels within a tube. A nanopipette that has a single channel within its tube is also called a "single-barreled nanopipette". A nanopipette having a plurality of channels within its tube is also called a "multi-barreled nanopipette". A nanopipette having two parallel channels within its tube is also called a "double-barreled nanopipette". The nanopipette used in the method of the present disclosure is preferably a single-barreled nanopipette from the perspective of ease and reliability of the operation of filling the nanopipette with a substance, and accuracy of current measurement and voltage application.

Nanopipettes are commercially available (for example, product number SU10ACC-NP01 manufactured by Yokogawa). Nanopipettes can also be made by, for example, drawing glass or quartz capillaries with a laser.

Details of the nanopipette are described in, for example, PTL 1 (WO 2014/160036 A1) and PTL 2 (WO 2013/012452 A1).

### (Three-dimensional (xyz) movement pipette holder)

The "three-dimensional (xyz) movement pipette holder" is a pipette holder to which a nanopipette is attached and which moves the attached nanopipette in three dimensions by driving a rough actuator and a fine actuator. The nanopipette is attached to the three-dimensional movement pipette holder so that two of the three directions (for convenience, also referred to as the "x-axis direction" and the "y-axis direction") are perpendicular or nearly perpendicular to the long axis of the nanopipette, and the remaining direction (for convenience, also referred to as the "z-axis direction") is parallel or nearly parallel to the long axis of the nanopipette. The three-dimensional movement pipette holder may be configured by, for example, a holder stage that is driven by the rough actuator and a holder head that is driven by the fine actuator, is mounted on the holder stage, and has the nanopipette attached thereto.

A "rough actuator" is a three-dimensional actuator that allows rough positioning, such as an actuator with a stroke on the order of 10 mm to 100 mm and a setting resolution on the order of 0.1 µm to 1 µm. Examples of rough actuators include electromagnetic force-driven actuators such as actuators based on motors (rotary motors, linear motors).

A "fine actuator" is a three-dimensional actuator that allows fine positioning, such a three-dimensional actuator with a stroke on the order of 100 µm to 500 µm and setting resolution on the order of 1 nm. Examples of fine actuators include piezoelectric effect driven actuators, such as piezoelectric element-based actuators. Furthermore, if the xy-axis setting resolution of the rough actuator has sufficient performance for the target cell size, the fine actuator may be specialized for precise approach to and penetration of cells and be limited to one dimension, i.e., only the z-axis.

### (Position input device)

The position input device is a device for manually inputting and indicating the position of the nanopipette. Examples of the position input device include a pointing device such as a joystick, a key input device such as a keyboard, and a combination of these.

### (Pipette electrode, reference electrode)

Examples of the pipette electrode and the reference electrode include a gold electrode, a silver electrode (such as a silver tetrakis(4-chlorophenyl)borate (AgTBACI) electrode or an Ag/AgCl electrode), a platinum electrode, and the like. The reference electrode may be used in contact with the electrolytic solution at the cell holder. Also, when the nanopipette is a multi-barreled (for example, double-barreled) nanopipette, the pipette electrode may be placed in a flow path that holds the substance to be introduced into the plant cell, and the reference electrode may be placed in another flow path.

### (Current measurement circuit, voltage application circuit)

The current measurement circuit is a circuit for measuring the ionic current between the pipette electrode and the reference electrode (i.e., the current between the inside of the nanopipette and the electrolytic solution). The current measurement circuit preferably has a current measurement range of the order of about 100 pA to 100 nA and is preferably capable of measuring a current change (reduction) of about 2 % to 50 % for cell surface detection. For example, in a case in which the current (steady state current) is 10 nA at a point sufficiently far from the cell surface, if detection of the cell surface is set as a 20 % decrease in current, the point at which the current becomes 8 nA is measured. Other examples include a low-noise amplifier circuit for accurately detecting a very small steady state reference current and changes in the current. Low noise may also be achieved using software-based digital filtering techniques.

The current measured by the current measurement circuit is used as an indicator of the distance between the cell and the tip of the nanopipette according to the principles of the scanning ion conductance microscopy (SICM) technique. When the tip of the nanopipette comes into contact with the electrolytic solution in the cell holder, an ionic current begins to flow between the tip and the reference electrode. Next, even if the tip of the nanopipette is moved closer to the cell, there is no significant change in the current value if the tip is sufficiently far from the cell surface. The current value at this time is called the "steady state current". Subsequently, upon the tip of the nanopipette coming extremely close to the cell, the current drops off rapidly according to the distance between the cell and the nanopipette tip. This is because the cell membrane is a highly insulating film. Therefore, by automatically controlling the nanopipette to temporarily stop when the drop rate of current from the steady state current reaches a preset value (hereinafter referred to as the "set current drop rate"), the nanopipette can be automatically paused, thereby automatically pausing the tip of the nanopipette immediately next to the cell.

### (Voltage application circuit)

The voltage application circuit is a circuit for applying a voltage between the pipette electrode and the reference electrode (between the inside of the nanopipette and the electrolytic solution). The voltage application circuit is preferably a circuit capable of applying a voltage on the order of -11 V to +11 V with time control on the order of 0.01 seconds.

### (Plant cells)

The methods of the present disclosure are applicable to plant cells. The form of the plant cells is not particularly limited and may be cultured cells, or cells present in callus, seeds, sprouts, plant tissue, plant tissue fragments, or the like. Examples of plant tissues include the shoot apical meristem of a sprout (for example, an L2 layer).

### (Cell holder)

The cell holder for holding the plant cells is not particularly limited, but typically a transparent holder with an openable top is used. Examples of such holders include cell culture vessels such as cell culture dishes and multi-well plates, the top of which is covered with an openable lid, and flat plates such as glass slides.

### (Cell immersion electrolytic solution)

Examples of the electrolytic solution in which the cells are immersed in the cell holder include liquid media (for example, Murashige and Skoog (MS medium), Gamborg B5 medium, Chu (N6) medium) and buffered saline (for example, phosphate buffered saline (PBS), HEPES buffered saline (HBS), Hank's balanced salt solution (HBSS), and the like).

### (Substance introduced into plant cell)

The substance to be introduced into the plant cell is not particularly limited, but examples thereof include proteins, mixtures or complexes containing proteins, mixtures or complexes containing proteins and nucleic acids, nucleic acids, and dyes. The substance to be introduced into the plant cell is not particularly limited but is preferably a substance that dissolves or is suspended in an electrolytic solution. The substance is also preferably an electrically charged substance. The electrically charged substance may be a single charged substance, a mixture or complex of plurality of substances that is charged as a whole, or a substance (single substance or a mixture or complex of a plurality of substances) that, when dissolved or suspended in an electrolytic solution, causes the solution as a whole to be charged. Electrically charged substances include positively charged substances and negatively charged substances. The substance to be introduced into the plant cell (for example, a protein, a mixture or complex containing a protein, a mixture or complex containing a protein and a nucleic acid, a nucleic acid, or a dye) is preferably either a positively charged substance or a negatively charged substance. For example, a protein, a mixture or complex of proteins, a mixture or complex containing nucleic acid and protein, or the like may be either a positively charged substance or a negatively charged substance. They may, for example, be a positively charged substance. An example of a mixture or complex containing a nucleic acid and a protein is a substance for genome editing. Substances for genome editing include CRISPR-Cas9, CRISPR-Cas3, ZFN, TALEN, and PPR systems. For example, nucleic acids (such as DNA or RNA) may be either a positively charged substance or a negatively charged substance. They may, for example, be a negatively charged substance. The substance introduced into the plant cell may be a marker substance such as a dye or may contain a marker substance.

The substance introduced into the plant cell is usually in the form of a liquid (for example, a solution). When the substance to be introduced into the plant cell is in the form of a solution, the solvent may be an aqueous electrolytic solution or a non-aqueous electrolytic solution, with an aqueous electrolytic solution being preferred. Examples of the aqueous electrolytic solution include those exemplified above as the electrolytic solution into which the cells are immersed.

### [Method for introducing substance into plant cell]

A method for introducing a substance into a plant cell according to the present disclosure includes the following steps.
a) positioning a nanopipette filled with a substance at a position corresponding to a plant cell in an electrolytic solution (pipette positioning step);
b) measuring a current between an inside of the nanopipette and the electrolytic solution and moving the nanopipette in a cell direction to a position at which a drop rate from a steady state current is a set current drop rate of 2 % or more and 50 % or less (pipette approach step);
c) moving the nanopipette in the cell direction by a set penetration distance of 1 µm or more and 50 µm or less to penetrate the plant cell (pipette penetration step);
d) applying a voltage between the inside of the nanopipette and the electrolytic solution to eject the substance into the plant cell (substance ejection step); and
e) retracting the nanopipette (pipette retraction step).

### (Step a): Pipette positioning step)

Step a) is a step of positioning a nanopipette filled with a substance at a position corresponding to a plant cell in the electrolytic solution. Methods of filling the inside of a nanopipette with a substance include, for example, a centrifugation method and a suction method. The centrifugation method may be performed by mounting the nanopipette in a centrifuge holder, filling the nanopipette with a substance through the base opening, and rotating the centrifuge holder with the nanopipette mounted thereon in a centrifuge. Suction may be accomplished by suctioning material from the tip opening. The nanopipette filled with the substance may be mounted, for example, in a three-dimensional (xyz) movement pipette holder as described above for positioning purposes. The "position corresponding to a plant cell" refers to a position on the "z-axis" from the position of the target plant cell. Positioning may be accomplished with a rough actuator, a fine actuator, or a combination of both. The rough actuator and the fine actuator may be driven by manual control via a position input device or automatically controlled by a program. Furthermore, another method is to position the xy-axes using a microscope stage.

### (Step b): Pipette approach step)

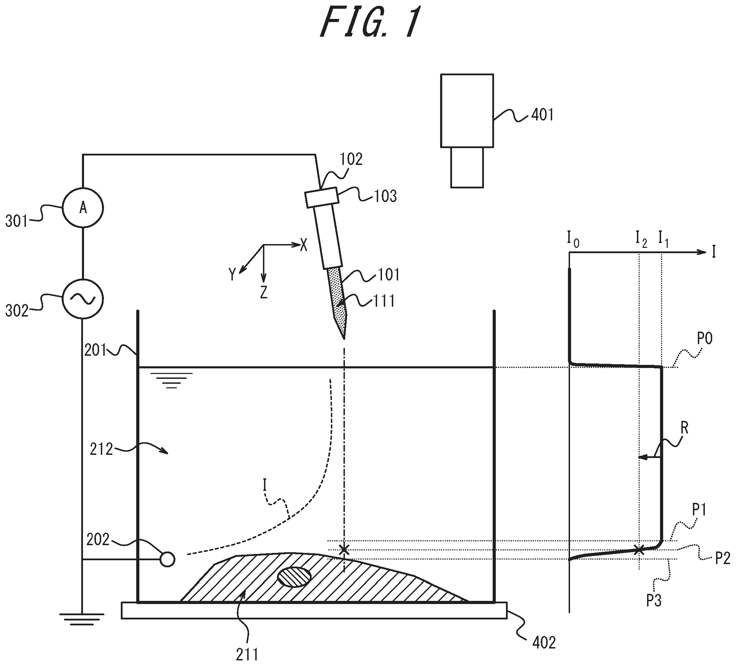

Step b) is a step of measuring the current between the inside of the nanopipette and the electrolytic solution and moving the nanopipette in the cell direction to a position at which the drop rate from the steady state current becomes the set current drop rate (the "pipette pause position (P2)" in FIG. 1). The "direction approaching the plant cell" refers to the direction toward the cell on the "z-axis". The current (ion current) between the inside of the nanopipette and the electrolytic solution can be measured by a current measurement circuit as the current between the pipette electrode and the reference electrode. In step b), in order to measure the current, it is preferable to apply a low voltage (set approach voltage) between the inside of the nanopipette and the electrolytic solution (i.e., between the pipette electrode and the reference electrode), the low voltage being set to a level that prevents the filling solution from flowing out due to electro-osmotic flow. The change in the current between the inside of the nanopipette and the electrolytic solution will be explained with reference to FIG. 1. When the tip of the nanopipette is located outside the electrolytic solution, the inside of the nanopipette is not connected to the electrolytic solution, and the current (I) becomes zero (I₀). When the tip of the nanopipette reaches the electrolytic solution surface position (P0) and enters the electrolytic solution, the inside of the nanopipette and the electrolytic solution are connected, and the current (I) increases. For a while thereafter, even if the nanopipette is advanced, the current (I) remains steady without any significant change. The current (I) at this time is defined as a steady state current (I1). When the tip of the nanopipette reaches a position ("current drop starting point position (P1)") extremely close to the cell surface position (P3), the current begins to drop rapidly according to the distance between the cell and the tip of the nanopipette. Thereafter, the nanopipette is advanced to a position ("pipette pause position (P2)") at which the current (I) becomes a current (I₂) that is reduced from the steady current (I₁) by a set current drop rate (R). The nanopipette may be paused at the pipette pause position (P2). The movement of the nanopipette in step b) may be accomplished with a rough actuator, a fine actuator, or a combination of both. At least the movement after the current drop starting point position (P1) is preferably performed by a fine actuator. The movement of the nanopipette in step b) is preferably performed automatically by a program. By presetting the set current drop rate according to the plant species, it is possible to suppress damage to the cells and achieve highly efficient introduction of substances into the cells.

### <Set current drop rate>

A value optimized for the plant cell is used as the set current drop rate. The set current drop rate is preferably 2 % to 50 %, more preferably 3 % to 40 %, and most preferably 5 % to 20 %. Furthermore, the set current drop rate can be optimized according to the plant species, as described in the "Examples of setting parameters" below.

### <Set approach voltage>

A value optimized for the plant cell is used as the set approach voltage. The value of the set approach voltage is preferably -2 V to +2 V, more preferably -1.0 V to +1.0 V. Furthermore, the set approach voltage can be optimized according to the plant species, as described in the "Examples of setting parameters" below.

### (Step c): Pipette penetration step)

Step c) is a step of moving the nanopipette from the aforementioned position (i.e., the pipette pause position (P2)) in the cell direction by a set penetration distance and penetrating the plant cell. "In the cell direction" refers to the direction towards the plant cell (the interior of the plant cell) on the "z-axis". The movement of the nanopipette during the pipette penetration step is usually performed by a fine actuator under automatic control by a program. The nanopipette is preferably moved at a higher speed in the pipette penetration step than in the pipette approach step. By presetting the penetration distance according to the plant species, it is possible to suppress damage to the cells and achieve highly efficient introduction of substances into the cells.

### <Set penetration distance>

A value optimized for the plant cell is used as the set penetration distance. The set penetration distance is preferably 1 µm to 50 µm, and more preferably 3 µm to 40 µm. Furthermore, the set penetration distance can be optimized according to the plant species, as described in the "Examples of setting parameters" below. When introducing a genome editing substance to obtain a genetically modified plant, a penetration distance that can reach the L2 layer of the shoot apical meristem is preferably set.

### (Step d): Substance ejection step)

Step d) is a step of applying a voltage between the inside of the nanopipette and the electrolytic solution to eject the substance into the plant cell. According to the type of substance being introduced into the plant cell (for example, a protein, a mixture or complex containing a protein, a mixture or complex containing a protein and a nucleic acid, a nucleic acid, a dye, or the like), a voltage is applied so that the inside of the nanopipette is at a positive potential and the electrolytic solution is at a negative potential, or alternatively, so that the inside of the nanopipette is at a negative potential and the electrolytic solution is at a positive potential. When the substance to be introduced into the plant cell is a positively charged substance, a voltage is applied so that the inside of the nanopipette is at a positive potential and the electrolytic solution is at a negative potential. When the substance to be introduced into the plant cell is a negatively charged substance, a voltage is applied so that the inside of the nanopipette is at a negative potential and the electrolytic solution is at a positive potential. The voltage is preferably applied at a set injection voltage (set applied voltage: V_{I}) and a set injection time (set application time: T₁).

### <Set injection voltage>

A value optimized for the plant cell is used as the set injection voltage. The set injection voltage is preferably -11 to +11 V, more preferably -10 to +10 V, and most preferably 4 V to 10 V, in the case of a positively charged substance. Furthermore, the set injection voltage can be optimized according to the plant species, as described in the "Examples of setting parameters" below.

### <Set injection time>

A value optimized for the plant cell is used as the set injection time. The set injection time is preferably 0.1 s to 10 s, more preferably 0.5 s to 5.0 s. Furthermore, the set injection time can be optimized according to the plant species, as described in the "Examples of setting parameters" below.

### (Step e): Pipette retraction step)

Step e) is a step of retracting the nanopipette. The "direction away from the plant cell" refers to the direction away from the plant cell (interior of the plant cell) on the "z-axis". The nanopipette movement may be performed at a set retraction distance. The movement of the nanopipette during the pipette retraction step is usually performed by a fine actuator under automatic control by a program.

### <Set retraction distance>

A value optimized for the plant cell is used as the set retraction distance. The set retraction distance is preferably within a range of 40 µm or more. The set puncture distance can be set to the above value for all plant species.

### [Method for producing a genetically modified plant or plant cell]

The method of the present disclosure can also be used as method for producing a genetically modified plant or plant cell. The method for producing a genetically modified plant or plant cell according to the present disclosure includes the following steps.
a) positioning a nanopipette filled with a genetic modification substance at a position corresponding to a plant cell in an electrolytic solution (pipette positioning step);
b) measuring a current between an inside of the nanopipette and the electrolytic solution and moving the nanopipette in a cell direction to a position at which a drop rate from a steady state current is a set current drop rate of 2 % or more and 50 % or less (pipette approach step);
c) moving the nanopipette in the cell direction by a set penetration distance of 1 µm or more and 50 µm or less to penetrate the plant cell (pipette penetration step);
d) applying a voltage between the inside of the nanopipette and the electrolytic solution to eject the substance into the plant cell (substance ejection step); and
e) retracting the nanopipette (pipette retraction step).

Genetic modification substances include positively charged genetic modification substances and negatively charged genetic modification substances. Examples of positively charged genetic modification substances include genetic modification substances based on mixtures or complexes containing proteins and nucleic acids, such as substances for genome editing (for example, Cas9-sgRNA-RNP complexes used in the CRISPR-Cas9 system, as well as substances used in genome editing systems such as CRISPR-Cas3, ZFN, TALEN, and PPR). Examples of negatively charged genetic modification substances include nucleic acids (DNA, RNA) and the like. Examples of nucleic acids include DNA, such as plasmid vectors, and RNA, such as antisense RNA and siRNA.

The conditions of the method for producing a genetically modified plant or plant cell can be the same as those described for the method for introducing a substance into a plant cell according to the present disclosure.

### [Control program for an apparatus for introducing a substance into a plant cell]

The present disclosure also provides a control program for an apparatus for introducing a substance into a plant cell. The program of the present disclosure includes instructions for performing the following steps.
a) positioning a nanopipette filled with a genetic modification substance at a position corresponding to a plant cell in an electrolytic solution (pipette positioning step);
b) measuring a current between an inside of the nanopipette and the electrolytic solution and moving the nanopipette in a cell direction to a position at which a drop rate from a steady state current is a set current drop rate of 2 % or more and 50 % or less (pipette approach step);
c) moving the nanopipette in the cell direction by a set penetration distance of 1 µm or more and 50 µm or less to penetrate the plant cell (pipette penetration step);
d) applying a voltage between the inside of the nanopipette and the electrolytic solution to eject the substance into the plant cell (substance ejection step); and
e) retracting the nanopipette (pipette retraction step).

The program of the present disclosure may further include instructions for controlling the approach voltage, the injection voltage (applied voltage), the injection time (application time), and the retraction distance.

The conditions controlled by the program of the present disclosure can be the same as those described for the method for introducing a substance into a plant cell according to the present disclosure.

### [Examples of setting parameters]

### [Example of settings parameters for introducing a positively charged substance]

### <Plants in general>

Approach voltage: preferably 0.5 V to 1.5 V, more preferably 0.8 V to 1.2 V, most preferably 1 V
Injection voltage: preferably 2 V to 11 V, more preferably 3 V to 10.5 V, most preferably 4 V to 10 V
Current drop rate: preferably 13 % to 21 %, more preferably 13.5 % to 20.5 %, most preferably 14 % to 20 %
Penetration distance: preferably 8 µm to 32 µm, more preferably 9 µm to 31 µm, most preferably 10 µm to 30 µm
Injection time: preferably 0.5 s to 6 s, more preferably 0.8 s to 6 s, most preferably 1 s to 5 s
Retraction distance: preferably 40 µm to 60 µm, more preferably 45 µm to 55 µm, most preferably 50 µm

### <Brassicaceae>

Approach voltage: preferably 0.5 V to 1.5 V, more preferably 0.8 V to 1.2 V, most preferably 1 V
Injection voltage: preferably 4 V to 11 V, more preferably 4.5 V to 10.5 V, most preferably 5 V to 10 V
Current drop rate: preferably 13 % to 21 %, more preferably 13.5 % to 20.5 %, most preferably 14 % to 20 %
Penetration distance: preferably 18 µm to 32 µm, more preferably 19 µm to 31 µm, most preferably 20 µm to 30 µm
Injection time: preferably 0.5 s to 5.5 s, more preferably 0.8 s to 5.2 s, most preferably 1 s to 5 s
Retraction distance: preferably 40 µm to 60 µm, more preferably 45 µm to 55 µm, most preferably 50 µm

### <<Raphanus>>

### - Example: Daikon (daikon sprouts) (Raphanus sativus var. hortensis)

Approach voltage: preferably 0.5 V to 1.5 V, more preferably 0.8 V to 1.2 V, most preferably 1 V
Injection voltage: preferably 4 V to 6 V, more preferably 4.5 V to 5.5 V, most preferably 5 V
Current drop rate: preferably 13 % to 21 %, more preferably 13.5 % to 20.5 %, most preferably 14 % to 20 %
Penetration distance: preferably 18 µm to 32 µm, more preferably 19 µm to 31 µm, most preferably 20 µm to 30 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### <Poaceae>

Approach voltage: preferably 0.5 V to 1.5 V, more preferably 0.8 V to 1.2 V, most preferably 1 V
Injection voltage: preferably 4 V to 11 V, more preferably 4.55 V to 10.5 V, most preferably 5 V to 10 V
Current drop rate: preferably 13 % to 21 %, more preferably 13.5 % to 20.5 %, most preferably 14 % to 20 %
Penetration distance: preferably 8 µm to 42 µm, more preferably 9 µm to 41 µm, most preferably 10 µm to 40 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### <<Hordeum>>

### Example: Barley (Hordeum vulgare)

Approach voltage: preferably 0.5 V to 1.5 V, more preferably 0.8 V to 1.2 V, most preferably 1 V
Injection voltage: preferably 2 V to 8 V, more preferably 3 V to 7 V, most preferably 4 V to 5 V
Current drop rate: preferably 13 % to 15 %, more preferably 13.5 % to 14.5 %, most preferably 14 %
Penetration distance: preferably 18 µm to 32 µm, more preferably 19 µm to 31 µm, most preferably 20 µm to 30 µm
Injection time: preferably 0.5 s to 6 s, more preferably 1 s to 6 s, most preferably 3 s to 5 s
Retraction distance: preferably 40 µm to 60 µm, more preferably 45 µm to 55 µm, most preferably 50 µm

### <<Zea>>

### - Example: Corn (Zea mays)

Approach voltage: preferably 0.5 V to 1.5 V, more preferably 0.8 V to 1.2 V, most preferably 1 V
Injection voltage: preferably 6 V to 11 V, more preferably 6.5 V to 10.5 V, most preferably 7 V to 10 V
Current drop rate: preferably 13 % to 21 %, more preferably 13.5 % to 20.5 %, most preferably 14 % to 20 %
Penetration distance: preferably 18 µm to 42 µm, more preferably 19 µm to 41 µm, most preferably 20 µm to 40 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### <Solanaceae>

### - Example: Solanum

Approach voltage: preferably 0.5 V to 1.5 V, more preferably 0.8 V to 1.2 V, most preferably 1 V
Injection voltage: preferably 4 V to 8 V, more preferably 4.5 V to 8.5 V, most preferably 5 V to 7 V
Current drop rate: preferably 13 % to 21 %, more preferably 13.5 % to 20.5 %, most preferably 14 % to 20 %
Penetration distance: preferably 8 µm to 32 µm, more preferably 9 µm to 31 µm, most preferably 10 µm to 30 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### - Example: Tomato (Solanum lycopersicum)

Approach voltage: preferably 0.5 V to 1.5 V, more preferably 0.8 V to 1.2 V, most preferably 1 V
Injection voltage: preferably 4 V to 8 V, more preferably 4.5 V to 8.5 V, most preferably 5 V to 7 V
Current drop rate: preferably 13 % to 15 %, more preferably 13.5 % to 14.5 %, most preferably 14 %
Penetration distance: preferably 18 µm to 32 µm, more preferably 19 µm to 31 µm, most preferably 20 µm to 30 µm
Injection time: preferably 2.5 s to 3.5 s, more preferably 2.8 s to 3.2 s, most preferably 3 s
Retraction distance: preferably 40 µm to 60 µm, more preferably 45 µm to 55 µm, most preferably 50 µm

### - Example: Potato (Solanum tuberosum)

Approach voltage: preferably 0.5 V to 1.5 V, more preferably 0.8 V to 1.2 V, most preferably 1 V
Injection voltage: preferably 4 V to 8 V, more preferably 4.5 V to 8.5 V, most preferably 5 V to 7 V
Current drop rate: preferably 19 % to 21 %, more preferably 19.5 % to 20.5 %, most preferably 20 %
Penetration distance: preferably 8 µm to 32 µm, more preferably 9 µm to 31 µm, most preferably 10 µm to 30 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### [Examples of setting parameters for introducing a negatively charged substance]

### <Plants in general>

Approach voltage: preferably -0.3 V to -0.7 V, more preferably -0.4 V to -0.6 V, most preferably -0.5 V
Injection voltage: preferably -2 V to -11 V, more preferably -2.5 V to -10.5 V, most preferably -3 V to -10 V
Current drop rate: preferably 9 % to 21 %, more preferably 9.5 % to 20.5 %, most preferably 10 % to 20 %
Penetration distance: preferably 8 µm to 32 µm, more preferably 9 µm to 31 µm, most preferably 10 µm to 30 µm
Injection time: preferably 0.3 s to 3.5 s, more preferably 0.4 s to 3.2 s, most preferably 0.5 s to 3 s
Retraction distance: preferably 40 µm to 60 µm, more preferably 45 µm to 55 µm, most preferably 50 µm

### <Brassicaceae>

### <<Example: Raphanus>>

### - Example: Daikon (daikon sprouts) (Raphanus sativus var. hortensis)

Approach voltage: preferably -0.3 V to -0.7 V, more preferably -0.4 V to -0.6 V, most preferably -0.5 V
Injection voltage: preferably -2 V to -8 V, more preferably -2.5 V to -7.5 V, most preferably -5 V to -7 V
Current drop rate: preferably 13 % to 21 %, more preferably 13.5 % to 20.5 %, most preferably 14 % to 20 %
Penetration distance: preferably 18 µm to 32 µm, more preferably 19 µm to 31 µm, most preferably 20 µm to 30 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### <Poaceae>

Approach voltage: preferably -0.3 V to -0.7 V, more preferably -0.4 V to -0.6 V, most preferably -0.5 V
Injection voltage: preferably -3 V to -8 V, more preferably -3.5 V to -7.5 V, most preferably -4 V to -7 V
Current drop rate: preferably 13 % to 15 %, more preferably 13.5 % to 14.5 %, most preferably 14 %
Penetration distance: preferably 18 µm to 32 µm, more preferably 19 µm to 31 µm, most preferably 20 µm to 30 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### <<Hordeum>>

### Example: Barley (Hordeum vulgare)

Approach voltage: preferably -0.3 V to -0.7 V, more preferably -0.4 V to -0.6 V, most preferably -0.5 V
Injection voltage: preferably -3 V to -8 V, more preferably -3.5 V to -7.5 V, most preferably -4 V to -7 V
Current drop rate: preferably 13 % to 15 %, more preferably 13.5 % to 14.5 %, most preferably 14 %
Penetration distance: preferably 18 µm to 32 µm, more preferably 19 µm to 31 µm, most preferably 20 µm to 30 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### <Solanaceae>

### - Example: Solanum

Approach voltage: preferably -0.3 V to -0.7 V, more preferably -0.4 V to -0.6 V, most preferably -0.5 V
Injection voltage: preferably -6 V to -11 V, more preferably -6.5 V to -10.5 V, most preferably -7 V to -10 V
Current drop rate: preferably 9 % to 15 %, more preferably 9.5 % to 14.5 %, most preferably 10 % to 14 %
Penetration distance: preferably 18 µm to 32 µm, more preferably 19 µm to 31 µm, most preferably 20 µm to 30 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### - Example: Tomato (Solanum lycopersicum)

Approach voltage: preferably -0.3 V to -0.7 V, more preferably -0.4 V to -0.6 V, most preferably -0.5 V
Injection voltage: preferably -6 V to -11 V, more preferably -6.5 V to -10.5 V, most preferably -7 V to -10 V
Current drop rate: preferably 13 % to 15 %, more preferably 13.5 % to 14.5 %, most preferably 14 %
Penetration distance: preferably 18 µm to 32 µm, more preferably 19 µm to 31 µm, most preferably 20 µm to 30 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### - Example: Potato (Solanum tuberosum)

Approach voltage: preferably -0.3 V to -0.7 V, more preferably -0.4 V to -0.6 V, most preferably -0.5 V
Injection voltage: preferably -6 V to -11 V, more preferably -6.5 V to -10.5 V, most preferably -7 V to -10 V
Current drop rate: preferably 9 % to 15 %, more preferably 9.5 % to 14.5 %, most preferably 10 % to 14 %
Penetration distance: preferably 18 µm to 32 µm, more preferably 19 µm to 31 µm, most preferably 20 µm to 30 µm
Injection time: preferably 0.5 s to 3.5 s, more preferably 0.8 s to 3.2 s, most preferably 1 s to 3 s
Retraction distance: preferably 40 µm to 60 µm, more preferably 45 µm to 55 µm, most preferably 50 µm

### EXAMPLES

The present disclosure will be described in more detail below using examples, but the technical scope of the present disclosure is not limited to these examples.

### (Preparation of nanopipette filled with reagent solution)

A nanopipette was prepared.

A genome editing tool reagent solution was prepared by dissolving the genome editing tool (Cas9-RNP) (10 ng/µL to 1 µg/µL) and a fluorescent reagent (FITC-labeled dextran) (10 µg/µL) in PBS.

Then, 3 µL or more of the reagent solution was taken up, using a micro loader attached to a centrifuge holder, and filled into the nanopipette from the top, and centrifuged for 30 s to 60 s in a tabletop centrifuge. The silver wire attached to the nanopipette was inserted through the hole at the top filled with the reagent solution and fixed with a special jig. At this time, it was confirmed that the silver wire was immersed in the reagent solution.

### (Plant sample preparation)

The plant species listed in Table 1 were used. The shoot apical meristem of the plant was exposed and arranged on a dish or the like. The individual plant was fixed using tape to prevent movement. Electrolyte solution was added so that the entire plant (especially the shoot apical meristem) was submerged.

### (Injection of reagent solution into cells of plant sample)

The plant sample was placed under a microscope. The nanopipette filled with the reagent solution was attached to the head of an apparatus for introducing a substance into plant cells (Single Cellome^{™} System UNIT SU10, manufactured by Yokogawa), and the rotary angle was adjusted. The reference electrode attached to the SU10 head was placed in the sample medium, and the SU10 software (measurement mode) was started. The nanopipette was brought into contact with the culture either manually or using the Liquid detect mode, and it was confirmed by software that the current value had increased from 0 nA. The SU10 joystick or the like was manipulated to position the nanopipette at a position corresponding to a target cell in the sample. The SU10 software was switched from measurement mode to delivery mode. Parameters were set according to the plant samples listed in Table 1. Start was pressed on the SU10 software to deliver the reagent solution into the target cell. Next, the joystick was operated for positioning at a position corresponding to the next target cell, and delivery was performed to the cells one after another. The sample after completion of delivery was removed with tweezers and transferred to a culture medium (an agar medium, a simple water-containing medium, soil, or the like). Thereafter, cultivation and growth of the plant were initiated.

**[Table 1]**

| Plant species | Daikon (Daikon sprouts) (Raphanus sativus var. hortensis) | Barley (Hordeum vulgare) | Tomato (Solanum lycopersicum) | Corn (Zea mays) | Potato (Solanum tuberosum) |
|---|---|---|---|---|---|
| Approach Voltage | Approach Voltage = 1V | Approach Voltage = 1V | Approach Voltage = 1V | Approach Voltage = 1V | Approach Voltage = 1V |
| Injection Voltage | Injection Voltage = 5V | Injection Voltage = 5V | Injection Voltage = 5 V to 7 V | Injection Voltage = 5 V to 10 V | Injection Voltage = 5 V to 7 V |
| Current Drop Rate | Current Drop Rate = 14 % to 20 % | Current Drop Rate = 14 % | Current Drop Rate = 14 % | Current Drop Rate = 14 % to 20 % | Current Drop Rate = 20 % |
| Penetration Distance | Penetration Distance = 20 µm to 30 µm | Penetration Distance = 20 µm to 30 µm | Penetration Distance = 20 µm to 30 µm | Penetration Distance = 20 µm to 40 µm | Penetration Distance = 10 µm to 30 µm |
| Injection Time | Injection Time = 1 s to 3 s | Injection Time = 1 s to 3 s | Injection Time = 3 s | Injection Time = 1 s to 3 s | Injection Time = 1 s to 3 s |
| Retraction Distance | Retraction Distance = 50 µm | Retraction Distance = 50 µm | Retraction Distance = 50 µm | Retraction Distance = 50 µm | Retraction Distance = 50 µm |

### (Genome editing tool introduction test into rice callus)

A genome editing tool (Cas9/gRNA complex: RNP) was introduced into rice callus using a substance introduction system (hereinafter referred to as the "SU10 system") formed by the same substance introduction device, nanopipette, microscope, and the like as described above. The target gene was the rice Phytoene desaturase gene (OsPDS). When this target gene is disrupted by genome editing, a phenotype involving the emergence of albino callus is exhibited. A plasmid carrying an expression cassette of a drug (hygromycin) resistance gene was co-introduced, and transformed callus was screened by performing selection culture.

### (Preparation of test rice callus)

Callus was induced from rice (Oryza sativa, cultivar: Nipponbare) seeds. The seeds were dehusked and sterilized using a solution of 70% ethanol and 10% Haiter^{®} (Haiter is a registered trademark in Japan, other countries, or both) (Kao). The sterilized seeds were rinsed with distilled water five or more times in a clean bench and then transplanted onto a callus induction medium (Table 2). The aseptically sown seeds were subjected to callus induction at 25 °C under dark conditions. Callus cultured for three weeks or longer was transferred to a fresh callus induction medium, whereby secondary callus was proliferated. Small, highly active callus was selected from the proliferated secondary callus and used in a state embedded in an agar medium.

**[Table 2]**

| **Rice callus induction medium** | |
|---|---|
| **Composition** | **Final concentration** |
| N6 inorganic salt | 1 |
| B5V | 1x |
| Sucrose | 30 mg/L |
| 2,4-D | 2 mg/L |
| Proline | 3 g |
| Casamino acids | 1 g |
| Gellan gum | 2 g/L |
| pH | 5.6 |

### (Preparation of genome editing tool (RNP))

In this test, genome editing was performed using the rice Phytoene desaturase (OsPDS) gene as a target. The base sequence of the gRNA used for genome editing (SEQ ID NO: 1) is illustrated in Table 3. The gRNA was synthesized using Guide-it CRISPR/Cas9 Systems (Takara Bio). The synthesized gRNA was complexed with Guide-it Recombinant Cas9 (Takara Bio) to prepare a genome editing tool (RNP) for use in the test. Complex formation was performed by incubating in 1x PBS buffer for 10 min or longer at room temperature.

**[Table 3]**

| **gRNA** sequence used for RNP synthesis | |
|---|---|
| **Target gene** | gRNA sequence (PAM sequence in parentheses) |
| **OsPDS gene (rice)** | GTTGGTCTTTGCTCCTGCAG(AGG) (SEQ ID NO: 1) |

### (Introduction and analysis of genome editing tool (RNP) using the SU10 system)

An injection test of the genome editing tool (RNP) into fixed rice callus was conducted using the SU10 system. The complexed RNP was loaded into a nanopipette at a concentration of 0.1 µM to 1 µM and mounted on the SU10 system. In addition, a test group in which a plasmid containing a selection marker was co-introduced at a concentration of 50 mg/L to 100 mg/L was also prepared. Treatment was performed on callus immobilized on a solid medium at a rate of 30 shots per callus. The buffer applied to the surface of the medium was MS medium, to which an antibacterial agent (Plant Preservative Mixture (PPM) (Plant Cell Technology)) was added.

For each RNP concentration group, delivery was performed on 2 to 5 calluses using the SU10 system under the conditions listed in Table 4. The treated calluses were incubated at 35 °C overnight to promote genome editing. Thereafter, the cells were cultured under normal callus induction conditions. After the recovery culture was completed, the cells were transferred to a drug (hygromycin) selection medium and sampled. DNA was extracted from the sampled calluses, and gene editing analysis was performed by next-generation sequencing (NGS).

I n Table 4, the injection parameter conditions (protocol) are as follows.
- Protein protocol
   App V = 1 V, Inj V = 4 V to 5 V, CDR = 14 %, Pen D = 20 µm, Inj T = 3 s to 5 s, Ret D = 50 µm
- Plasmid protocol
   App V = -1 V, Inj V = -4 V, CDR = 14 %, Pen D = 20 µm, Inj T = 3 s, Ret D = 50 µm

**[Table 4]**

| Test No. | Plasmid concentration (mg/L) | RNP concentration (µM) | Number of introductions | Number of calluses | Introduction protocol | Number of selected calluses |
|---|---|---|---|---|---|---|
| 1 | 100 | | 30 | 2 | Plasmid | 1 |
| 2 | 100 | 1 | 30 | 2 | Protein | 1 |
| 3 | | 1 | 30 | 2 | Protein | |
| 4 | 10 | | 30 | 5 | Plasmid | |
| 5 | 50 | | 30 | 5 | Plasmid | 2 |
| 6 | 100 | | 30 | 5 | Plasmid | 1 |
| 7 | 100 | 0.1 | 30 | 5 | Protein | 1 |
| 8 | 100 | 0.5 | 30 | 5 | Protein | 2 |
| 9 | 100 | 1 | 30 | 5 | Protein | 1 |
| 10 | | 0.1 | 30 | 5 | Protein | |
| 11 | | 0.5 | 30 | 5 | Protein | |
| 12 | | 1 | 30 | 5 | Protein | |
| 13 | 10 | | 30 | 5 | Plasmid | |
| 14 | 50 | | 30 | 5 | Plasmid | 1 |
| 15 | 100 | | 30 | 5 | Plasmid | 1 |
| 16 | 100 | 0.1 | 30 | 5 | Protein | 2 |
| 17 | 100 | 0.5 | 30 | 5 | Protein | 1 |
| 18 | 100 | 1 | 30 | 5 | Protein | 1 |
| 19 | 100 | 0.5 | 100 | 2 | Protein | |
| 20 | 100 | 1 | 100 | 2 | Protein | |

### (Results of the test to introduce genome editing tool into rice callus)

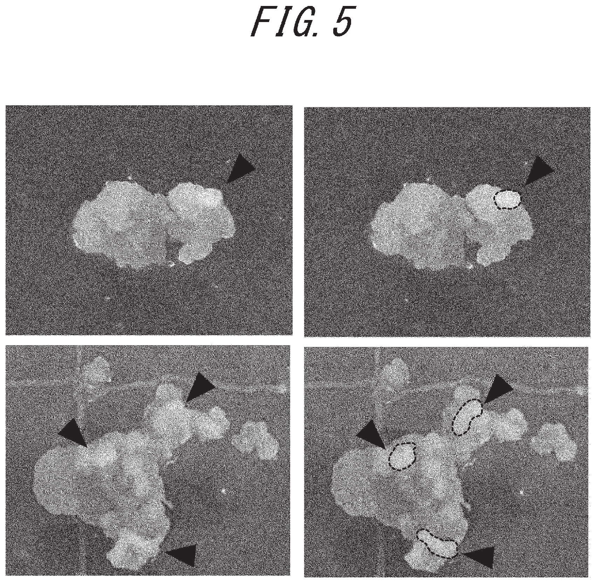

Drug selection culture was performed on a drug (hygromycin) selection medium, and drug-resistant callus was obtained as callus in which plasmid delivery into plant cells had been successful ("number of selected calluses" in Table 4). It was confirmed that the introduction of the DNA-free genome editing tool (RNP) resulted in the appearance of callus exhibiting a partially white phenotype caused by the target gene PDS mutation. Photographs of calluses (two samples) exhibiting a partially albino phenotype in test group #17 are depicted in FIG. 5.

In addition, genetic analysis (NGS) confirmed that the introduction of the DNA-free genome editing tool (RNP) resulted in changes in the base sequence of the target gene PDS mutation. The mutation (SEQ ID NO: 3) appeared at a rate of about 2 % compared to the wild type (SEQ ID NO: 2). It was confirmed that sequence changes caused by genome editing occurred at a site slightly shifted from the theoretical mutation site (cut site). Reanalysis confirmed the same mutation, thus confirming a definite, albeit off-target, mutation.

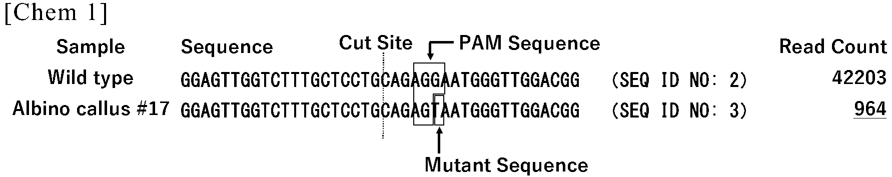

### (Test to introduce genome editing tool into barley growth point)

The SU10 system was used to introduce a genome editing tool (Cas9/gRNA complex: RNP) into the seed growth point of barley (Hordeum vulgare). The target gene was the barley Phytoene desaturase gene (HvPDS). When this target gene is disrupted by genome editing, a phenotype involving the emergence of an albino portion is exhibited.

To perform CAPS analysis of edited individuals, a gRNA sequence on HvPDS was identified. A gRNA sequence (SEQ ID NO: 4, Table 5) was designed on an exon of HvPDS, and the gRNA was complexed with a Cas9 protein to form a complex (RNP), which was then introduced into the barley growth point using the SU10 system. The introduced individuals were subjected to recovery culture for about 2 days and were then cultivated in cell trays.

**[Table 5]**

| **gRNA sequence used for RNP synthesis** | |
|---|---|
| **Target gene** | gRNA sequence (PAM sequence in parentheses) |
| **HvPDS** | GTACGACCTCCCTTGGCTTA(AGG) (SEQ ID NO: 4) |

Specifically, each step was carried out as follows.

### 1. Selection and sequencing of gRNA candidates

A gRNA sequence within HvPDS (LOC123449634) was identified based on information obtained from a barley database. Candidate gRNAs were located on exons, and eight sequences having restriction enzyme sites near the Cas9 cleavage site (the third nucleotide from the PAM sequence) were selected as candidate sequences. In addition, gRNA candidates were further narrowed down based on sequence analysis of the barley seeds actually used. The selected gRNAs were synthesized using Guide-it CRISPR/Cas9 Systems (Takara Bio), complexed with a Cas9 protein to form an RNP, and subjected to an in vitro cleavage activity assay. As a result, the candidate gRNA sequence to be used was determined to be SEQ ID NO: 4 (Table 5).

### 2. Preparation of introduced RNP and introduction using SU10

The determined gRNA was mixed with Cas9 protein to synthesize RNP. The RNP was prepared at a concentration of 1 µM. Complex formation was performed using a mixed buffer of 1x PBS buffer by incubation at room temperature for 10 min or longer. The synthesized RNP was introduced into 30 cells of the growth point of barley seeds using the SU10 system after exposing the growth point of the barley seeds that had been watered overnight.

### 3. Cultivation of introduced barley seeds

The RNP-introduced seeds were cultivated on an MS medium. Since roots grew within 1 or 2 days after cultivation, the seeds were transferred onto a Kimtowel (Nippon Paper Crecia) containing sterilized water in order to suppress the occurrence of bacteria and mold. The next day, the seedlings were transplanted into cell trays filled with soil containing vermiculite and nursery soil at a ratio of 1:1 and were cultivated.

### (Results of the test to introduce genome editing tool into barley shoot growth point)

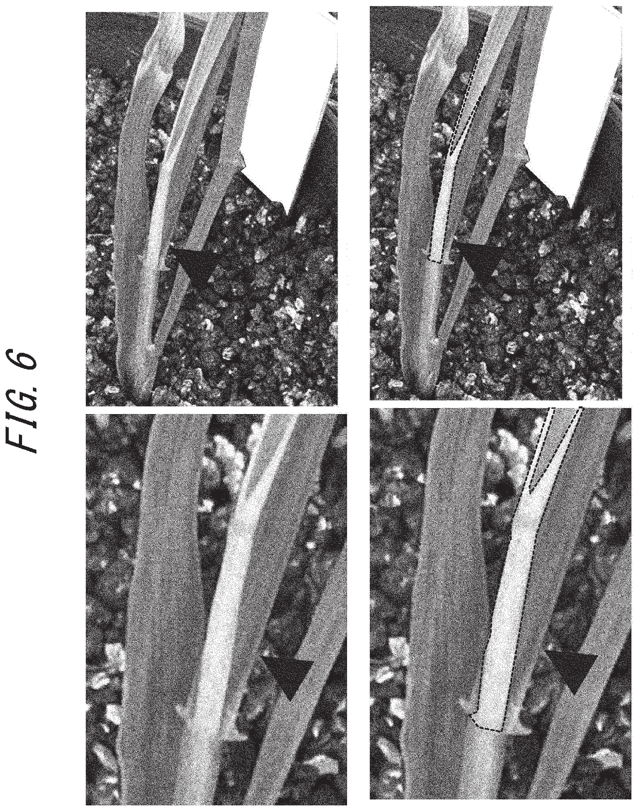

After introduction of the genome editing tool into the barley growth point (shoot apical meristem), it was confirmed that individuals exhibiting whitening at the central portion of the stem appeared after normal growth and development to whole plants (FIG. 6). This whitening was the phenotype observed in PDS gene mutations caused by genome editing.

### (Search for optimal parameters for direct injection into individual plants by test to inject reagent solution into the growth point of barley)

To establish parameters for performing genome editing with high efficiency, optimal parameters for direct injection into individual plants were investigated. In searching for optimal parameters, the index of optimization was set to be the ability to deliver the reagent solution to plants with high efficiency and to ensure the survival rate of the plants after introduction.
Target sample: Barley (Hordeum vulgare)
Delivery substance: GFP-protein (reagent by Abcam)
Number of deliveries = 10 times per individual
N number = 2 (however, for injection voltage 5 V and time 3 s to 5 s, N number = 4)
Microscope: Stereo microscope (SZX10 by Evident)
Nanopipette: NP02 (manufactured by Yokogawa Electric Corporation)

Optimal conditions were narrowed down with respect to combinations of injection voltage and injection time, which were considered to affect delivery efficiency, as parameters.

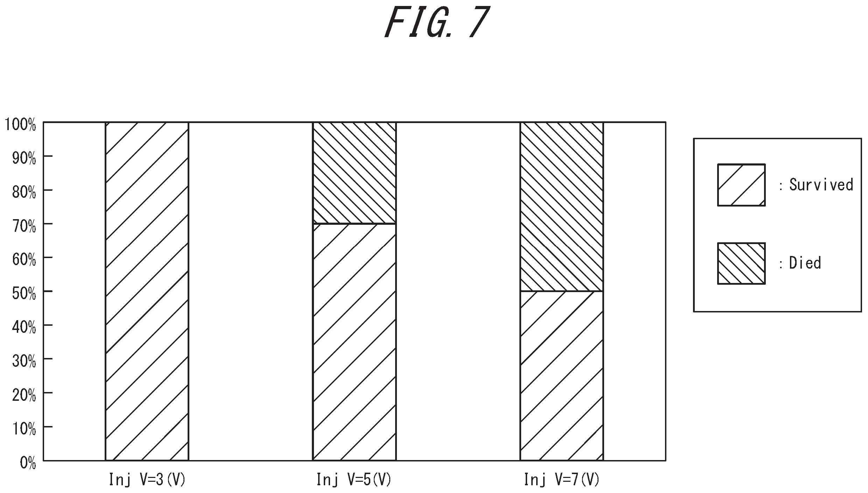

A good success rate of substance introduction was confirmed under conditions of an injection voltage of 3 V to 7 V and an injection time of 3 s to 5 s. It was confirmed that the success rate of introduction per individual was particularly high, with a maximum of 50 % when the injection voltage was 5 V and the application time was 3 s, and a maximum of 70 % when the injection voltage was 5 V and the injection time was 5 s. On the other hand, a tendency for the delivery success rate to decrease was confirmed when the injection voltage was 2 V or less and 8 V or more. This suggested that the amount of substance delivered per cell may depend on the injection voltage and injection time. The results of the delivery success rate are listed in Table 6. The results of the survival rate of the plants after introduction are illustrated in FIG. 7.

**[Table 6]**

| | **2 V** | **3 V** | **5 V** | **7 V** | **8 V** | **9 V** |
|---|---|---|---|---|---|---|
| **3 s** | 10 % | 10 % | 35 % | 7 % | 7 % | 10 % |
| **5 s** | 5 % | 10 % | 15 % | 25 % | 7 % | 7 % |
| **6 s** | - | 15 % | 20 % | 10 % | 10 % | 5 % |

| | | | | | | |
|---|---|---|---|---|---|---|
| (the "-" indicates that data was not acquired) | | | | | | |

Target sample: Barley (Hordeum vulgare)
Delivery substance: FITC dextran
Number of deliveries = 10 times per individual
N number = 3
Microscope: Stereo microscope (SZX10 by Evident)
Nanopipette: NP02 (manufactured by Yokogawa Electric Corporation)

Optimal conditions were narrowed down with respect to the set current drop rate, which was considered to affect delivery efficiency, as a parameter.

A good substance introduction success rate was confirmed when the set current drop rate (CDR) was 3 % or more and 40 % or less, and an even better substance introduction success rate was confirmed when the set current reduction rate was 5 % or more and 20 % or less. The results of the delivery success rate are listed in Table 7. The success rate of substance introduction was defined as the percentage for which fluorescence was observed immediately after introduction among a total (30) of 10 introductions each of a fluorescent reagent (FITC-dextran) into the shoot apical meristem of individual barley (N = 3). The success rate of substance introduction was evaluated as follows.
⊚: 20 % or more
△: 10 % or more and less than 20 %
×: Less than 10 %

**[Table 7]**

| CDR | 50 % | 45 % | 40 % | 35 % | 20 % |
|---|---|---|---|---|---|
| Evaluation | × | × | Δ | Δ | ⊚ |
| Success rate of substance introduction | 0% | 3% | 10 % | 13 % | 30 % or more |

| CDR | 1 % | 3 % | 5 % |
|---|---|---|---|
| Evaluation | × | ⊚ | ⊚ |
| Success rate of substance introduction | 3 % | 20 % | 30 % |

### INDUSTRIAL APPLICABILITY

According to the present disclosure, injection conditions specific to plants can be established in a method for injecting into a plant cell using a nanopipette, and an automatically controlled injection system for plant cells can be provided.

### REFERENCE SIGNS LIST

- 101: Nanopipette
- 102: Pipette electrode
- 103: Three-dimensional (xyz) movement pipette holder
- 111: Substance introduced into plant cell
- 201: Cell holder
- 202: Reference electrode
- 211: Plant cell
- 212: Electrolytic solution
- 301: Current measurement circuit
- 302: Voltage application circuit
- 401: Optical microscope observation portion
- 402: Optical microscope stage
- D: Set penetration distance
- I: Ion current
- I₀: Base current
- I₁: Steady state current
- I₂: Current decreased at set current drop rate
- P0: Electrolytic solution surface position
- P1: Current drop starting point position
- P2: Pipette pause position
- P3: Cell surface position
- P4: Material discharge position
- R: Set current drop rate
- T: Time
- T₁: Injection time (application time)
- V: Voltage
- V_{A}: Approach voltage
- V_{I}: Injection voltage (applied voltage)

## Claims

1. A method for introducing a substance into a plant cell, the method comprising:
a) positioning a nanopipette filled with a substance at a position corresponding to a plant cell in an electrolytic solution;
b) measuring a current between an inside of the nanopipette and the electrolytic solution and moving the nanopipette in a cell direction to a position at which a drop rate from a steady state current is a set current drop rate of 2 % or more and 50 % or less;
c) moving the nanopipette in the cell direction by a set penetration distance of 1 µm or more and 50 µm or less to penetrate the plant cell;
d) applying a voltage between the inside of the nanopipette and the electrolytic solution to eject the substance into the plant cell; and
e) retracting the nanopipette.

2. The method according to claim 1, wherein the substance is a protein, a mixture or complex containing a protein, a mixture or complex containing a protein and a nucleic acid, a nucleic acid, or a dye.

3. The method according to claim 2, wherein the substance is a substance for genome editing.

4. The method according to claim 2, wherein
the substance is a positively charged substance or a negatively charged substance,
in a case in which the substance is a positively charged substance, d) is performed by applying a voltage so that the inside of the nanopipette is at a positive potential and the electrolytic solution is at a negative potential, and
in a case in which the substance is a negatively charged substance, d) is performed by applying a voltage so that the inside of the nanopipette is at a negative potential and the electrolytic solution is at a positive potential.

5. The method according to claim 4, wherein d) is performed by applying a voltage at a set applied voltage of -11 V or more and +11 V or less and a set application time of 0.1 seconds or more and 10.0 seconds or less.

6. A method for producing a genetically modified plant or plant cell, the method comprising:
a) positioning a nanopipette filled with a genetic modification substance at a position corresponding to a plant cell in an electrolytic solution;
b) measuring a current between an inside of the nanopipette and the electrolytic solution and moving the nanopipette in a cell direction to a position at which a drop rate from a steady state current is a set current drop rate of 2 % or more and 50 % or less;
c) moving the nanopipette in the cell direction by a set penetration distance of 1 µm or more and 50 µm or less to penetrate the plant cell;
d) applying a voltage between the inside of the nanopipette and the electrolytic solution to eject the substance into the plant cell; and
e) retracting the nanopipette.

7. A genetically modified plant or plant cell produced by a method comprising:
a) positioning a nanopipette filled with a genetic modification substance at a position corresponding to a plant cell in an electrolytic solution;
b) measuring a current between an inside of the nanopipette and the electrolytic solution and moving the nanopipette in a cell direction to a position at which a drop rate from a steady state current is a set current drop rate of 2 % or more and 50 % or less;
c) moving the nanopipette in the cell direction by a set penetration distance of 1 µm or more and 50 µm or less to penetrate the plant cell;
d) applying a voltage between the inside of the nanopipette and the electrolytic solution to eject the substance into the plant cell; and
e) retracting the nanopipette.

8. A control program for an apparatus for introducing a substance into a plant cell, the control program comprising instructions for executing processing comprising:
a) positioning a nanopipette filled with a genetic modification substance at a position corresponding to a plant cell in an electrolytic solution;
b) measuring a current between an inside of the nanopipette and the electrolytic solution and moving the nanopipette in a cell direction to a position at which a drop rate from a steady state current is a set current drop rate of 2 % or more and 50 % or less;
c) moving the nanopipette in the cell direction by a set penetration distance of 1 µm or more and 50 µm or less to penetrate the plant cell;
d) applying a voltage between the inside of the nanopipette and the electrolytic solution to eject the substance into the plant cell; and
e) retracting the nanopipette.
